Europäisches Patentamt

European Patent Office

Office européen des brevets

(19)

(11) Numéro de publication : **0 615 757 A1**

# (12) DEMANDE DE BREVET EUROPEEN

(21) Numéro de dépôt : **94400584.2**

(22) Date de dépôt : **17.03.94**

(51) Int. Cl.⁵ : **A61K 35/72,** A61K 33/30,
A61K 33/04, A61K 31/195,
A61K 31/07

(30) Priorité : **18.03.93 FR 9303138**

(43) Date de publication de la demande :
**21.09.94 Bulletin 94/38**

(84) Etats contractants désignés :
**BE CH DE ES GB GR IE IT LI LU NL PT**

(71) Demandeur : **Grimberg, Georges Serge**
**123 rue de l'Université**
**F-75007 Paris (FR)**

(72) Inventeur : **Grimberg, Georges Serge**
**123 rue de l'Université**
**F-75007 Paris (FR)**

(74) Mandataire : **Madeuf, Claude Alexandre Jean**
**et al**
**CABINET MADEUF**
**3, avenue Bugeaud**
**F-75116 Paris (FR)**

(54) **Composition thérapeutique nouvelle ayant un effet immunostimulant.**

(57)    Composition pharmaceutique nouvelle ayant une activité immunostimulante, caractérisée en ce qu'elle se compose de quatre groupes de produits : une vitamine, du soufre, une cellule biologique complexe, un cation.

EP 0 615 757 A1

Lorsqu'on étudie l'activité immunostimulante, c'est-à-dire la capacité donnée à l'organisme de se défendre contre une attaque bactérienne ou virale, on constate que différentes substances peuvent avoir une action.

La présente invention consiste à associer différents produits et notamment une vitamine, du soufre, une cellule biologique complexe, un cation.

Conformément à l'invention, la composition contient :
- de la vitamine A,
- du soufre organique : la L-Cystine, et
- du soufre minéral : le soufre sublimé

la cellule biologique complexe étant de la levure qui contient elle-même une foule de vitamines, de protéines, d'oligo-éléments, etc. Le cation est du zinc.

Suivant une autre caractéristique de l'invention, chaque gélule contient :
- vitamine A          20 000 UI
- soufre sublimé          22 mg
- L-Cystine          72,6 mg
- levure          77,4 mg
- sulfate de zinc          100 mg

Diverses autres caractéristiques de l'invention ressortent d'ailleurs de la description détaillée qui suit.

Au cours des essais cliniques, les deux produits suivants (gélule rouge et gélule verte) ont été testés pour leur activité immunostimulante dans le cadre de la réponse anticorps primaire post-vaccinale anti-hépatite B.

TABLEAU I

|  | Gélule rouge | Gélule verte | Gélule jaune |
|---|---|---|---|
| Vitamine A Acétate | 20 000 UI | 20 000 UI | 0 |
| Soufre sublimé lavé | 22 mg | 22 mg | 0 |
| L Cystine base | 72,6 mg | 72,6 mg | 0 |
| Levure | 77,4 mg | 77,4 mg | 0 |
| Sulfate de Zinc 7H O | 0 | 100 mg | 0 |
| Cellulose micro-cristalline | 0 | 0 | 137,7 mg |

L'étude a été réalisée en double aveugle contre placebo (gélule jaune), au sein d'une population d'élèves gendarmes.

L'étude a utilisé le vaccin Hevac B Pasteur. L'antigène viral a été injecté trois fois à un mois d'intervalle.

Cette vaccination a été faite après un examen sanguin qui démontrait l'absence d'antigène HBs, l'absence d'anticorps anti HBs et anti HBc.

Le critère de jugement a été immunobiologique après chacune des injections. On a mesuré le nombre de séro convertis, c'est-à-dire les sujets dont le taux d'anticorps anti HBs était supérieur à 10 UI/L, et de la même manière les taux des séro convertis.

Chaque élève gendarme a donc été vacciné une fois par mois pendant trois mois, et a pris en double aveugle ou une gélule jaune quatre fois par jour, ou une gélule rouge quatre fois par jour, ou une gélule verte quatre fois par jour.

L'analyse statistique démontre au deuxième mois une indéniable activité immunostimulante et pour la gélule verte un résultat statistiquement significatif.

TABLEAU II

|  | Gélule jaune | Gélule rouge | Gélule verte |
|---|---|---|---|
| Nombre de séro convertis | 14 | 13 | 19 |
| Moyenne des anticorps | 43 | 226 | 531 |

Le rapport des taux moyens des anticorps ainsi obtenu entre la gélule verte et la gélule jaune est supérieur à 12. De plus, ce rapport est statistiquement significatif ($P<0,05$).

Il faut bien comprendre que, dans le cas présent, les jeunes qui ont été vaccinés ont bien répondu au vaccin car le nombre de séro convertis est voisin dans les trois groupes mais, en revanche, l'activité immunostimulante est forte dans le groupe gélule rouge et très forte dans le groupe gélule verte.

En résumé, l'association Vitamine A, L-Cystine, soufre sublimé lavé, levure morte, a une activité immunostimulante.

Mais, lorsqu'on ajoute un cation comme le zinc par exemple, cette activité est très nettement augmentée.

Le zinc est connu pour son activité in vivo sur certaines réactions biologiques bien déterminées mais différents cations le sont aussi et peuvent donc eux aussi être associés à cette formule.

Le cation zinc peut être du sulfate de zinc, du gluconate de zinc ou tout autre sel de zinc pharmacologiquement acceptable. De plus, à la place du zinc, on peut employer du cuivre, du manganèse, du fer ou d'autres métaux et cela en fonction du virus ou de la bactérie.

Il est bien évident que la quantité de chacun des principes actifs de cette association (vitamine, soufre, cellule biologique complexe, cation) peut être diminuée ou augmentée dans la gamme d'au moins 1 à 3, en fonction de l'effet immunostimulant désiré.

## Revendications

**1** - Composition pharmaceutique nouvelle ayant une activité immunostimulante, caractérisée en ce qu'elle se compose de quatre groupes de produits : une vitamine, du soufre, une cellule biologique complexe, un cation.

**2** - Composition pharmaceutique nouvelle selon la revendication 1, caractérisée en ce que la vitamine est de la vitamine A, le soufre est sous forme organique de la L-Cystine, et minéral du soufre sublimé lavé, la cellule biologique est de la levure ; le cation est du zinc.

**3** - Composition pharmaceutique suivant l'une des revendications 1 et 2, caractérisée en ce que les quantités de chacun des principes actifs peuvent être variables de 1 à 3 et même plus, en fonction de l'effet immunostimulant désiré.

**4** - Composition pharmaceutique suivant l'une des revendications 1, 2 et 3, caractérisée en ce que le cation zinc peut être du sulfate de zinc, du gluconate de zinc ou tout autre sel de zinc.

**5** - Composition pharmaceutique suivant l'une des revendications 1, 2, 3 et 4, caractérisée en ce que le cation zinc peut être remplacé par un autre cation dont on connaît l'activité sur certaines réactions biologiques.

**6** - Composition thérapeutique nouvelle ayant un effet immunostimulant sensiblement telle que décrite.

Office européen
des brevets

**RAPPORT DE RECHERCHE EUROPEENNE**

Numero de la demande

EP 94 40 0584

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| X | CD-ROM VIDAL 1992, O.V.P. - EDITIONS DU VIDAL, PARIS. <br> * SOLACY adulte * <br> --- | 1-6 | A61K35/72 <br> A61K33/30 <br> A61K33/04 <br> A61K31/195 <br> A61K31/07 |
| X | CD-ROM VIDAL 1992, O.V.P. - EDITIONS DU VIDAL, PARIS. <br> * SOLACY pédiatrique * <br> --- | 1-6 | |
| Y | FR-A-2 228 470 (M.R.R. OUDOT) <br> * le document en entier * <br> --- | 1-6 | |
| Y | FR-A-2 431 863 (H.A. MARTIN) <br> * le document en entier * <br> --- | 1-6 | |
| Y | EP-A-0 402 208 (G.S. GRIMBERG) <br> * le document en entier * <br> --- | 1-6 | |
| Y | EP-A-0 113 608 (G.S. GRIMBERG) <br> * le document en entier * <br> --- | 1-6 | |
| Y | US-A-4 428 933 (J.R. KING) <br> * revendications * <br> --- | 1-6 | **DOMAINES TECHNIQUES RECHERCHES (Int.Cl.5)** <br><br> A61K |
| Y | EP-A-0 530 060 (G.S. GRIMBERG) <br> * le document en entier * <br> ----- | 1-6 | |

Le présent rapport a été établi pour toutes les revendications

| Lieu de la recherche | Date d'achèvement de la recherche | Examinateur |
|---|---|---|
| LA HAYE | 18 Mai 1994 | Orviz Diaz, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

&......................................................................
& : membre de la même famille, document correspondant

EPO FORM 1503 03.82 (P04C02)